# EUROPÄISCHE PATENTSCHRIFT

(11) **EP 2 311 427 B1**
(45) Veröffentlichungstag und Bekanntmachung des Hinweises auf die Patenterteilung: **29.07.2015**
(21) Anmeldenummer: 10013716.5
(22) Anmeldetag: 18.10.2010
(51) Int. Cl.: A61N 7/00

(54) **Ultraschallbehandlungsgerät und Verfahren zu dessen Betrieb**
Ultrasound treatment device and method for its operation
Appareil de traitement à ultrasons et son procédé de fonctionnement

(30) Priorität: 16.10.2009 DE 102009049795
(43) Veröffentlichungstag der Anmeldung: 20.04.2011
(73) Patentinhaber: Becker, Reinhard, 34538 Bad Karlshafen (DE); Helgert, Erich, 82538 Geretsreid (DE)
(72) Erfinder: Becker, Reinhard, 34538 Bad Karlshafen (DE); Helgert, Erich, 82538 Geretsreid (DE)
(74) Vertreter: Schneider, Peter Christian

(56) Entgegenhaltungen:
- KR-B1- 100 773 119
- US-A- 5 425 704
- US-A- 5 524 624
- US-A1- 2006 149 169
- US-A1- 2008 173 729
- US-A1- 2009 254 008
- US-B1- 6 183 426

## Beschreibung

### Gebiet der Erfindung

Die Erfindung betrifft ein Ultraschallbehandlungsgerät mit einer in einem Gehäuse angeordneten Energieversorgung, einem Oszillator und einem dem Oszillator nachgeschalteten Treiber mit nachfolgender Leistungsstufe, die mit einem Ultraschall-Wandlerelement eines Schallkopfes verbunden ist.

Die Erfindung betrifft weiterhin ein Verfahren zum Betrieb eines Ultraschallbehandlungsgerätes, bei dem ein Ultraschall-Wandlerelement eines Schallkopfes von einem Oszillatorsignal eines Oszillators über einen nachgeschalteten Treiber erregt wird.

### Stand der Technik

Ultraschall im Bereich von ca. 1 MHz erzeugt im Wasser Kavitation (Blasenbildung). Dieser Effekt wird zunehmend für medizinische und kosmetische Behandlungen der Haut genutzt. Hierbei können Wirkstoffe besser in die Haut eingebracht werden. Sobald der Ultraschall in die Haut eindringt, entsteht im vorhandenen Wasser bzw. Flüssigkeit der Haut Kavitation, wodurch der Wirkstoff besser eingeschleust werden kann.

Aus der DE 40 15 686 A1 ist ein Ultraschallbehandlungsgerät für Ultraschallfrequenzen oberhalb von 100 KHz bekannt. Das Gerät weist in einem Gehäuse neben einer Energieversorgung einen Oszillator, einen dem Oszillator nachgeschalteten Treiber und eine nachfolgende Leistungsstufe auf, die mit einem Ultraschall-Wandlerelement des Schallkopfes verbunden ist. Der Oszillator weist dabei eine Oszillatorfrequenz auf, die mit Eigenfrequenz des Ultraschall-Wandlerelementes übereinstimmen soll, um maximale Schwingungsamplituden und eine entsprechend große abgestrahlte Ultraschall-Schwingungsenergie zu erhalten.

Nachteilig dabei ist, dass die verwendeten Ultraschall-Wandlerelemente produktionsbedingt eine Streuung ihrer Resonanzfrequenz aufweisen. Zusätzlich ändern auch Wärmeinflüsse oder in Kontakt stehende weitere Materialien diese Frequenz.

Weiterhin ist aus der DE 10 2008 009 468 A1 eine Vorrichtung und ein Verfahren zur Durchblutungssteigerung und Verbesserung des Sauerstoffaustausches bekannt, bei dem eine kosmetische Zubereitung auf die zu behandelnde Haut appliziert wird und anschließend mithilfe eines Ultraschallapplikators in die zu behandelnde Haut eingearbeitet wird oder dass eine kosmetische Zubereitung auf einen Ultraschallapplikator aufgetragen wird und die zu behandelnde Haut anschließend mithilfe des Ultraschallapplikators mit Ultraschall behandelt wird. Dieses bekannte Ultraschallbehandlungsgerät weist ebenfalls die oben beschriebenen Nachteile auf.

Weiterhin ist aus der CH 446 783 ein Ultraschallgenerator mit einem Ultraschall-Resonanz-Schwingungssystem für industrielle Anwendungen, wie Ultraschallschweißen, Pressen, Bearbeiten u.s.w. bekannt. Bei diesem System wird der Ultraschallgenerator durch eine magnetorstriktive Rückkopplung automatisch auf die Resonanzfrequenz des Schwingungssystems abgestimmt. Dabei ist es notwendig, dass das Ultraschall-Übertragungselement aus magnetostriktiven Material ausgebildet ist. Derartige Geräte sind relativ voluminös und aufwendig ausgebildet und somit nicht zur Verwendung für kleine kompakte Handgeräte geeignet. Zudem ist eine magnetorstriktive Rückkupplung nicht für ein Ultraschall-Wandlerelement geeignet, das als ein Piezoelement ausgebildet ist.

Aus der US 6 183 426 B1 ist ein Ultraschallbehandlungsgerät mit einer in einem Gehäuse angeordneten Energieversorgung, einem Oszillator, der als ein von einem Mikrocontroller gesteuerter programmierbarer Oszillator ausgebildet ist, bekannt. In Verbindung mit einer Strommessung zur Ansteuerung des Ultraschall-Wandlerelements ist bei einem maximalen Stromfluss eine optimale Resonanzfrequenz ermittelbar, mit der der Oszillator programmiert und das Wandlerelement betrieben wird.

Nachteilig dabei ist, dass sich bei unterschiedlichen Impedanzen die abgestrahlte Schallleistung verändert und nicht konstant bleibt. Bei dem bekannten Ultraschallbehandlungsgerät ist eine Lastdetektionsschaltung vorgesehen, die überwacht, ob das Schwingelement z.B. durch Kontakt mit der Haut belastet ist und ein entsprechendes Detektionssignal liefert. Weiterhin ist eine Bewegungsdetektionsschaltung vorgesehen, die überwacht, ob sich der Ultraschallkopf bewegt und ein Bewegungsdetektionssignal liefert, wenn der Ultraschallkopf sich bewegt. Bei kritischen Lastdetektionssignalen oder Bewegungsdetektionssignalen wird von einer Steuerschaltung die Ultraschallleistung reduziert. Eine konstante Regelung der abgegebenen Schallleistung findet bei dem bekannten Ultraschallbehandlungsgerät nicht statt.

Aus der US 5 524 624 A ist ein von einem Mikrocontroller gesteuerter Oszillator bekannt, der über ein ebenfalls von dem Mikrocontroller gesteuertes Gate über einen Treiber mit einem Ultraschallwandler verbunden ist. Der dem Treiber aus einer Stromquelle zugeführte Stromfluss wird dabei offensichtlich nicht gemessen.

Die Ermittlung einer optimalen Resonanzfrequenz ist dieser Druckschrift nicht zu entnehmen. Der Fachmann erhält aus dieser Druckschrift auch keinen Hinweis, den dem Treiber zugeführten Stromfluss durch eine Puls-Pausen-Modulation des Gates auf einem vorgegebenen, gegenüber dem maximalen Stromwert reduzierten Strom-Soll-Wert konstant zu halten.

Aus der US 2008/173729 A1 ist ein Ultraschallvernebler bekannt, der ebenfalls einen von einem Mikroprozessor gesteuerten Oszillator aufweist, der über einen Verstärker mit einem Ultraschall-Wandlerelement verbunden ist. Zur Ermittlung der Resonanzfrequenz des Ultraschall-Wandlerelements wird ein Frequenzscan durchgeführt, bei dem die Resonanzfrequenz in Abhängigkeit von der Impedanz des Ultraschall-Wandlerelements ermittelt wird. Im Resonanzfall ergibt sich in bekannter Weise ein maximaler Stromwert. Die gefundene Resonanzfrequenz wird in einem Speicher des Mikroprozessors gespeichert und der Oszillator wird mit der gefundenen Resonanzfrequenz betrieben.

Eine Regelung der Schallleistung ist aus der D3 nicht bekannt.

Auch aus der KR 100 773 119 B1 ist es bekannt, einen Frequenzscan in Verbindung mit einer Strommessung durchzuführen, um die Resonanzfrequenz eines Ultraschallwandlers zu ermitteln.

Der Anmeldungsgegenstand weist die oben aufgeführten Vorteile auf und ist daher auch gegenüber der D4 erfinderisch.

### Aufgabenstellung

Aufgabe der vorliegenden Erfindung ist es daher, ein handliches und kompaktes Ultraschallbehandlungsgerät anzugeben, dass mit einer optimalen Resonanzfrequenz bei möglichst konstanter Schallleistung betreibbar ist.

Weitere Aufgabe der Erfindung ist es, ein Verfahren zum Betrieb eines Ultraschallbehandlungsgerätes anzugeben, dass die oben genannten Nachteile nicht aufweist.

### Darstellung der Erfindung

Die Aufgabe betreffend des Ultraschallbehandlungsgerätes wird in Verbindung mit dem Oberbegriff des Anspruches 1 dadurch gelöst, dass die Leistungsstufe als ein Filter ausgebildet ist, über den die Rechteckspannung des Treibers in eine sinusförmige Betriebsspannung umwandelbar und die Impedanz des Treibers an die Impedanz des Ultraschall-Wandlerelements anpassbar ist, dass der Oszillator als ein von einem Mikrocontroller gesteuerter programmierbarer Oszillator ausgebildet ist, dass in Verbindung mit einer Strommessung, die den dem Treiber zugeführten Stromfluss misst, zur Ansteuerung des Ultraschall-Wandlerelements eine optimale Resonanzfrequenz bei maximalem Stromfluss ermittelbar ist und dass die ermittelten Werte in einem Speicher abspeicherbar und von dem Mikrocontroller verarbeitbar sind und dass zur Leistungsregelung durch eine Puls-Pausen-Modulation zwischen dem programmierbaren Oszillator und dem Treiber ein von dem Mikrocontroller ansteuerbares Gate angeordnet ist, wobei ein gegenüber dem maximalen Stromwert reduzierter Stromsollwert zur Leistungsregelung konstant gehalten werden kann.

Durch die Ausbildung des Oszillators als programmierbarer Oszillator können über den Mikrocontroller verschiedene Oszillatorfrequenzen eingestellt und eine den Oszillatorfrequenzen zuordenbare Strommessung durchgeführt werden. Dabei kann ein maximaler Strom zur Ansteuerung des Ultraschall-Wandlerelements gemessen und seine zugehörige Frequenz ermittelt werden. Dadurch ist es möglich, die optimale Resonanzfrequenz, die bei einem maximalen Stromfluss auftritt, als Arbeitsfrequenz zu ermitteln und den Oszillator mit der als optimale Resonanzfrequenz ermittelten Frequenz zu programmieren. Der Mikroprozessor regelt dabei die Arbeitsfrequenz über den programmierbaren Oszillator, der eine Rechteckspannung ausgibt, deren Leistung durch den dem Oszillator nachgeschalteten Treiber erhöht wird. Die dem Treiber nachgeschaltete Leistungsstufe als ein Filter ausgebildet, über den die Rechteckspannung des Treibers in eine sinusförmige Betriebsspannung umwandelbar und die Impedanz des Treibers an die Impedanz des Ultraschall-Wandlerelementes anpassbar ist. Durch die Anpassung der Impedanz wird Energie eingespart. Die Betriebsspannung wird für die Erzeugung des Ultraschalls über das Ultraschall-Wandlerelement genutzt, das beispielsweise als ein Piezoelement ausgebildet ist.

Zur Leistungsregelung durch eine Puls-Pausen-Modulation ist zwischen dem programmierbaren Oszillator und dem Treiber ein von dem Mikrocontroller ansteuerbares Gate angeordnet. Dadurch ist es möglich, die Schallleistung bei wechselnden Impedanzen konstant zu halten. Da sich einzelne Ultraschall-Wandlerelemente bzw. Piezoelemente in der Serie unterscheiden, kann der maximale Stromfluss von Ultraschall-Wandlerelement zu Ultraschall-Wandlerelement variieren. Bei der Puls-Pausen-Modulation wird das Gate so ein- und ausgeschaltet, dass im Mittel immer eine festgelegte und konstante Leistung abgegeben wird. Wird beispielsweise die Impedanz des Schallkopfes erhöht (durch z.B. Körperkontakt) und die Schallleistung sinkt, kann auf diesem Wege der Strom erhöht werden, damit die Ultraschallleistung konstant bleibt.

Gemäß einer bevorzugten Ausführungsform der Erfindung ist das Gehäuse als ein rohrförmiges Handstück ausgebildet, das zu einer Applikationsstelle, also zur Haut des Patienten hin, von dem Schallkopf verschlossen ist. An der Innenseite des Schallkopfes ist das Ultraschall-Wandlerelement angeordnet. Das Ultraschall-Wandlerelement ist beispielsweise als ein scheibenförmiges Piezoelement ausgebildet, das an der Innenseite der Stirnwandung des Schallkopfes mit diesem verklebt ist. Dabei ist der Schallkopf als eine Art metallische Kappe ausgebildet, die der chemischen Abgrenzung des Piezokristalls bzw. des Ultraschall-Wandlerelementes zur Umgebung dient. Die Stärke der Stirnwandung ist dabei so berechnet, dass sowenig wie möglich Ultraschallleistung, beispielsweise durch Interferenzen innerhalb des Metalls, in ihr absorbiert wird. Gleichzeitig soll möglichst viel Ultraschall abgegeben werden. Als Material können beispielsweise Aluminium aber auch Titan oder Keramiken verwendet werden. Damit die Schallwellen nicht in das rohrförmige Handstück gelangen, weist die Stirnwandung des Schallkopfes an der Innenseite eine umlaufende Nut auf.

Gemäß einer weiteren bevorzugten Ausführungsform der Erfindung weist das Gehäuse an seinem dem Schallkopf abgewandten Ende einen das Gehäuse verschließenden Touch-Schalter auf. Insbesondere durch einen Touch-Schalter lässt sich das Gehäuse an seinem der dem Schallkopf abgewandten Ende leicht und sicher abdichten. Der Touch-Schalter kann auch an einer das Gehäuse verschließenden Endkappe angeordnet sein.

Nach einer weiteren bevorzugten Ausführungsform der Erfindung ist im Gehäuse ein Temperatursensor vorgesehen, wobei bei Überschreiten einer vorgebbaren Grenztemperatur die Leistung des Schallkopfes reduzierbar ist. Dadurch kann eine zu starke Erwärmung des Gerätes im eingeschalteten Zustand, insbesondere bei längerer Nichtbenutzung, verhindert werden. Bei Bedarf kann dann über die Puls-Pausen-Modulation die Leistung des Schallkopfes reduziert werden.

Aufgrund der verwandten hocheffizienten Elektronik ist eine starke Miniaturisierung möglich. Dadurch kann die Elektronik direkt im Handgriff integriert werden, wodurch der Weg zum Schallgeber bzw. Ultraschall-Wandlerelement sehr kurz gehalten werden kann und somit eine Hochfrequenzzuleitung, wie sie bei Standgeräten verwendet werden muss, nicht notwendig ist.

Die Aufgabe betreffend das Verfahren zum Betrieb eines Ultraschallbehandlungsgerätes wird in Verbindung mit dem Oberbegriff des Anspruches 9 dadurch gelöst, dass der Oszillator von einem Mikrocontroller gesteuert und programmiert wird, dass zwischen dem programmierbaren Oszillator und dem Treiber ein von dem Mikrocontroller ansteuerbares Gate angeordnet ist, wobei nach dem Einschalten des Ultraschallbehandlungsgerätes folgende Schritte durchgeführt werden:
a) Frequenzscan, bei dem ein vorgegebenes Frequenzband bei gleichzeitiger Messung des Stromflusses, der dem Treiber zugeführt wird, durchfahren wird, wobei die ermittelten Werte in einem Arbeitsspeicher abgelegt werden,
b) Ermittlung des Maximums des Stromflusses und zugehöriger optimaler Resonanzfrequenz,
c) Betrieb des Ultraschall-Wandlerelements mit der ermittelten optimalen Resonanzfrequenz bei einer Regelung der Schallleistung durch Puls-Pausen-Modulation des Gates auf einen vorgegebenen konstanten Wert, wobei der dem Treiber zugeführte Stromfluss durch die Puls-Pausen-Modulation auf einem vorgegebenen, gegenüber dem maximalen Stromwert reduzierten Stromsollwert konstant gehalten wird.

Bei dem Frequenzscan und der gleichzeitigen Messung des Stromflusses können auf relativ einfache und sichere Weise Frequenzen und zugehörigen Stromwerte ermittelt und in einem Arbeitsspeicher abgelegt werden. Anschließend können die gemessenen Werte aus dem Arbeitsspeicher ausgelesen und von dem Mikrocontroller zur Ermittlung des Maximums des Stromflusses und zugehöriger optimaler Resonanzfrequenz als Arbeitsfrequenz ausgewertet werden. Bei dem Einschalten führt das Gerät selbstständig den Frequenzscan durch. Hierbei wird ein Frequenzband, welches sich über dem eigentlichen Arbeitspunkt des Schallgebers erstreckt, mittels des programmierbaren Oszillators durchfahren. Zu den jeweiligen Frequenzen wird der Stromfluss über den Schallkopf gemessen und im Arbeitsspeicher des Gerätes abgelegt. Sobald der Scan durchgeführt ist, wird das Maximum des Stromflusses ermittelt und der Schallgeber bzw. das Ultraschall-Wandlerelement wird mit dieser Frequenz betrieben. Der zur optimalen Resonanzfrequenz ermittelte maximale Stromwert wird durch eine Puls-Pausen-Modulation eines zwischen dem Oszillator und einem nachfolgenden Treiber angeordneten Gates auf einen vorgegebenen Wert reduziert und damit die Leistung geregelt. Dadurch wird weniger Energie für den Betrieb benötigt.

Gemäß einer weiteren bevorzugten Ausführungsform der Erfindung werden die Schritte a) bis c) in vorgegebenen Zyklen wiederholt. Dies ist sinnvoll, da durch physikalische Änderungen, wie Erwärmungen des schallgebenden Ultraschall-Wandlerelements, Kontakt zu anderen Medien etc., eine Verschiebung der Resonanzfrequenz eintritt. Somit ist sichergestellt, dass immer mit der entsprechenden Resonanzfrequenz gearbeitet wird und das Ultraschallbehandlungsgerät eine reproduzierbare Schallleistung abgibt.

Gemäß einer weiteren bevorzugten Ausführungsform der Erfindung wird als optimale Resonanzfrequenz eine unterhalb der maximalen Resonanzfrequenz liegende Frequenz verwendet.

Gemäß einer weiteren bevorzugten Ausführungsform der Erfindung wird über einen Temperatursensor die Temperatur des Schallkopfes ermittelt und bei Überschreiten einer vorgegebenen Grenztemperatur die Leistung des Schallkopfes reduziert. Dadurch kann effektiv eine zu starke Erwärmung des Gerätes im eingeschalteten Zustand bei längerer Nichtbenutzung verhindert werden.

Die Schallköpfe weisen eine Resonanzfrequenz oberhalb von 100 kHz auf, bevorzugt etwa 1 MHz oder höher.

Weitere Einzelheiten der Erfindung ergeben sich aus der nachfolgenden ausführlichen Beschreibung und den beigefügten Zeichnungen, in denen bevorzugte Ausführungsformen der Erfindung beispielhaft veranschaulicht sind.

### Kurzbeschreibung der Zeichnungen

In den Zeichnungen zeigen:

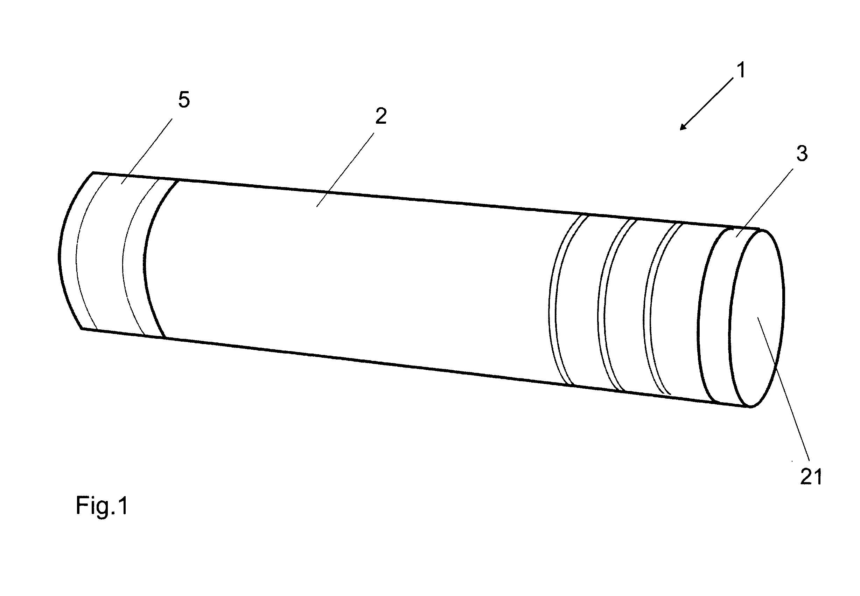

- Figur 1:: eine räumliche Darstellung eines Schallkopfes mit eingeklebtem Ultraschall-Wandlerelement als Schallgeber.

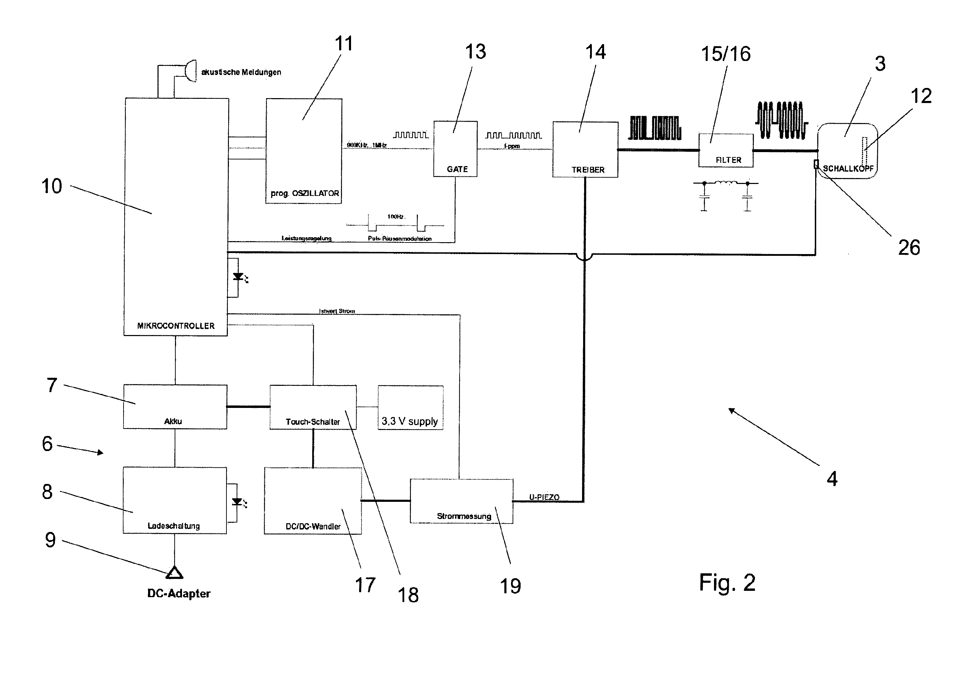

- Figur 2:: die elektronische Schaltung des Ultraschallbehandlungsgerätes als Blockschaltbild,
- Figur 3:: einen Stromverlauf des dem Schallkopf zugeführten Stromes in Abhängigkeit von der Zeit,
- Figur 4:: den Stromverlauf in Abhängigkeit von der Zeit vor, während und nach einem Frequenzscan,
- Figur 5:: eine Seitenansicht im Schnitt eines Schallkopfes mit eingeklebtem Ultraschall-Wandlerelement und
- Figur 6:: einen weiteren Stromverlauf in Abhängigkeit von der Zeit während eines Frequenzscans.

### Beschreibung der Ausführungsbeispiele

Ein Ultraschallbehandlungsgerät 1 besteht im Wesentlichen aus einem Gehäuse 2 mit einem Schallkopf 3, einer elektrischen Schaltung 4 und einer Endkappe 5.

Die elektrische Schaltung 4 weist eine Energieversorgung 6 auf, die im Wesentlichen aus einem Akkumulator 7, einer vorgeschalteten Ladeschaltung 8 und einem DC-Adapter 9 besteht. Die Energieversorgung 6 speist einen nachgeschalteten Mikrocontroller 10, der als Mikroprozessor die Steuer- und Regeleinheit des Ultraschallbehandlungsgerätes 1 bildet. Der Mikrocontroller 10 steuert einen programmierbaren Oszillator 11, mit dem er die Arbeitsfrequenz des Schallkopfes 3 bzw. des in dem Schallkopf 3 angeordneten Ultraschall-Wandlerelements, das den eigentlichen Schallgeber darstellt, erzeugt. Das Ultraschall-Wandlerelement 12 ist als ein Piezoelement ausgebildet, welches die Form einer runden Scheibe aufweist und für die Erzeugung von Ultraschall im Bereich von 1 MHz ausgelegt ist. Die von dem programmierbaren Oszillator 11 abgegebene Rechteckspannung wird einem Gate 13 zugeführt, das ebenfalls mit dem Mikrocontroller verbunden ist und über das durch eine Puls-Pausen-Modulation eine Leistungsregelung durchgeführt werden kann. Mithilfe der Puls-Pausen-Modulation des Gates 13 ist es möglich, die Schallleistung bei wechselnden Impedanzen konstant zu halten. Ein nachfolgender Treiber 14, der über die Energieversorgung 6 gespeist wird, erhöht die Leistung der von dem Oszillator 11 über das Gate 13 ausgegebenen Rechteckspannung. Eine nachfolgende Leistungsstufe 15, die als ein spezieller Filter 16 ausgebildet ist, erzeugt aus der Rechteckspannung des Treibers 14 eine sinusförmige Betriebsspannung zum Betrieb des Ultraschall-Wandlerelementes 12. Dabei wird zusätzlich die Impedanz des Treibers 14 der Impedanz des Ultraschall-Wandlerelementes 12 angepasst. Ein DC/DC-Wandler 17 der Energieversorgung 6 ist über einen Touch-Schalter 18 mit dem Akkumulator 7 verbunden. Der DC/DC-Wandler 17 führt über eine Strommessung 19 dem Treiber 14 Energie zu. Die Strommessung 19 steht zur Weiterleitung des gemessenen Strom-Ist-Wertes mit dem Mikrocontroller 10 in Verbindung.

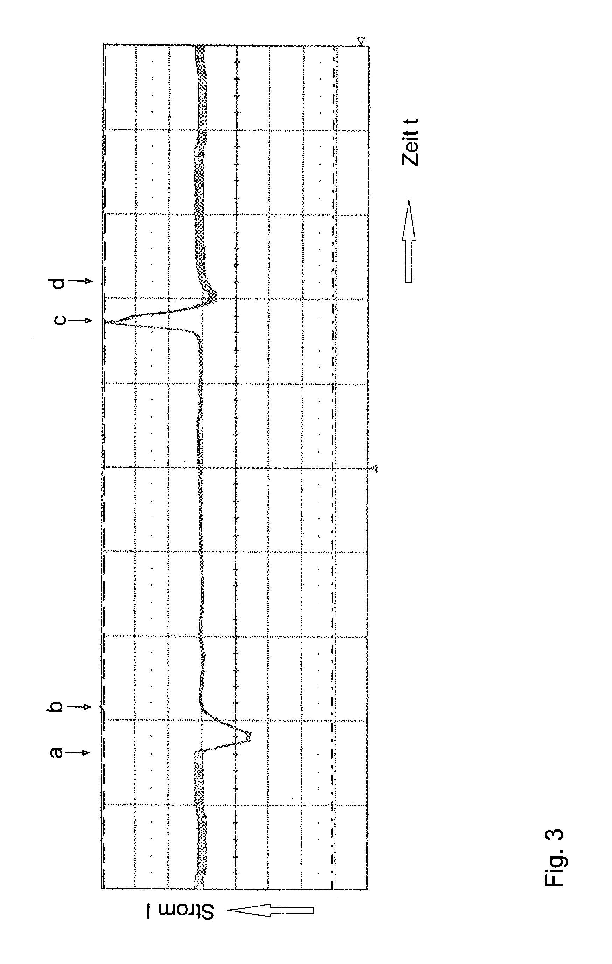

In Figur 3 ist der von der Strommessung 19 ermittelte IstWert-Strom über die Zeit aufgetragen. Bis Punkt a liegt am Schallkopf 3 keine Last an, d.h. der Schallkopf 3 bzw. das Ultraschall-Wandlerelement 12 schwingt an Luft, der Strom ist konstant. Bei Punkt a wird die Erhöhung der Impedanz, d.h. durch das Aufbringen einer Last/Dämpfung am Schallkopf 3, die Leistung verringert, so dass der Strom absinkt. Die Elektronik bzw. der Mikrocontroller 10 erkennt dies und erhöht den Strom I, bis der vorgegebene Wert wieder erreicht ist (siehe Punkt b). Sobald der Schallkopf 3 wieder frei von der Last ist und frei schwingen kann, erhöht sich der Strom (siehe Punkt c). Dabei wird der Strom I von dem Mikrocontroller 10 mithilfe der Puls-Pausen-Modulation über das Gate 13 wieder auf den vorgegebenen Soll-Wert reduziert (siehe Punkt d).

Nach dem Einschalten des Ultraschallbehandlungsgerätes 1 über den Touch-Schalter 18 werden folgende Schritte durchgeführt:
a) es wird ein Frequenzscan in einem vorgegebenen Frequenzband, beispielsweise zwischen 900 KHz und 1 MHz, bei gleichzeitiger Messung des Stromflusses, der über den Treiber 14 dem Ultraschall-Wandlerelement 12 zugeführt wird, durchfahren, wobei die ermittelten Werte in einem Arbeitsspeicher abgelegt werden,
b) Ermittlung des Maximums des Stromflusses und zugehöriger optimaler Resonanzfrequenz,
c) Betrieb des Ultraschall-Wandlerelements 12 mit der ermittelten optimalen Resonanzfrequenz.

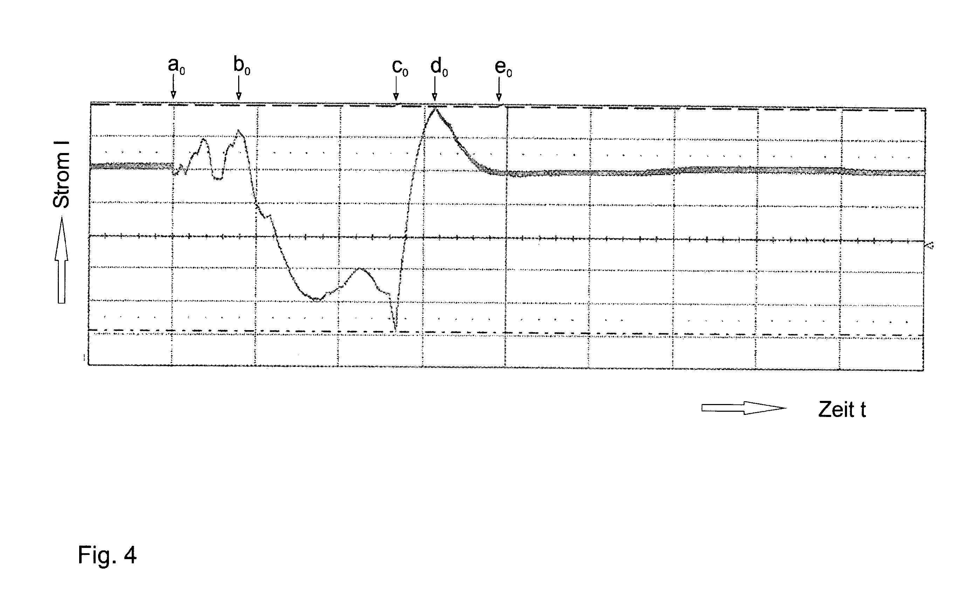

Figur 4 zeigt den Stromverlauf über der Zeit vor, während und nach Verlauf des Frequenzscans. Bis Punkt a₀ liegt die für den Standardbetrieb genutzte Resonanzfrequenz an. Ab Punkt a₀ beginnt der Scan von einer vorgegebenen niedrigen Frequenz, beispielsweise 920 KHz, bis zum einer definierten hohen Frequenz C₀ beispielsweise 1 MHz. Zu den jeweiligen Frequenzen wird der Strom gemessen und ist im Diagramm der Figur 4 dargestellt. Bei Punkt b₀ liegt das Strommaximum vor. Diese Frequenz ist ab Punkt c₀ eingestellt. Bei Punkt b₀ ist der maximale Strom erreicht, der durch die Puls-Pausen-Modulation (PPM) auf den benötigten und voreingestellten Wert reduziert wird. Damit wird sichergestellt, dass immer bei der Resonanzfrequenz des Schallkopfes 3 gearbeitet und die definierte bzw. vorgegebene Leistung als Ultraschall ausgegeben wird.

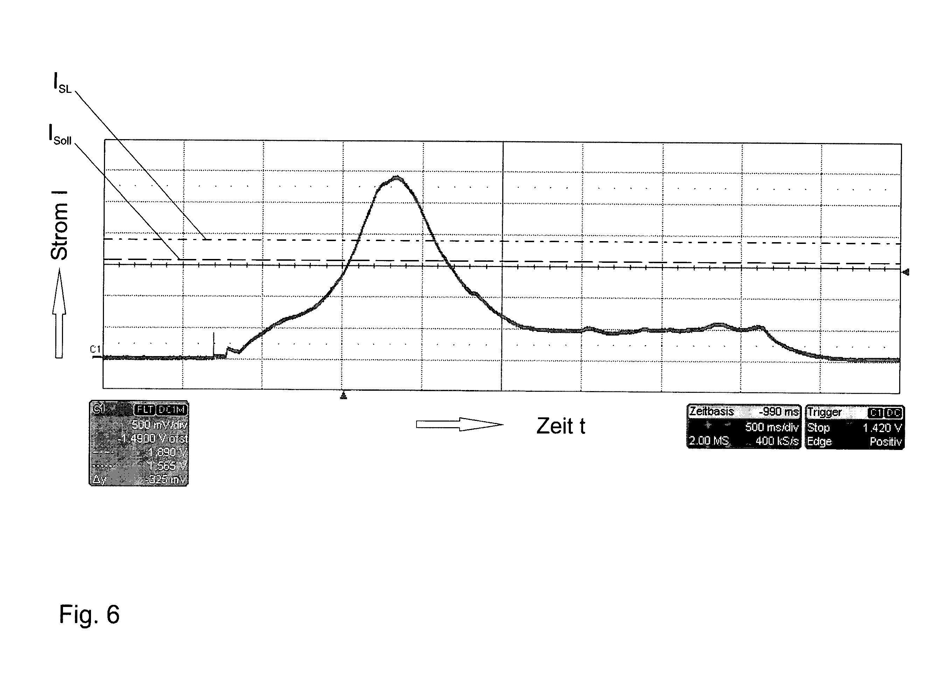

Nach einem weiteren Ausführungsbeispiel zeigt Figur 6 den Verlauf der Stromkurve nach dem Einschalten des Ultraschallgerätes während eines Analysescan. Er dient dazu, die Frequenz festzustellen, bei der die Stromkurve die strichpunktierte Linie als Scanlimit I_{SL} schneidet, dieser Punkt bzw. das Scanlimit I_{SL} ist in der Software eine voreinstellbare Variable. Die Frequenzinkremente betragen im Beispiel 500 Hz. Der Scanbereich liegt bei ca. 910 kHz bis ca. 980 kHz. In der Folge wird diese Frequenz als Oszillatoreinstellung festgehalten und mittels PPM (100 Hz) der Strom so eingestellt, dass der Stromwert als Stromsollwert I_{Soll} (gestrichelte Linie) als ebenfalls voreingestellte Variable konstant gehalten wird, und somit unabhängig von den Dämpfungen des Schallkopfs wird. Die Schallleistung bleibt dadurch praktisch konstant. Dabei ist es für eine Konstanthaltung der Schallleistung von Vorteil, als optimale Resonanzfrequenz eine etwas unterhalb der maximalen Resonanzfrequenz liegende Frequenz zu verwenden.

Die technische Grundidee des Ultraschallgerätes basiert auf der konstanten Schallleistung des Schallgebers. D.h. dass die Leistung entsprechend der vorliegenden Dämpfung nachgeregelt wird. Hierzu wird die Impedanz in Abhängigkeit von der Frequenz gemessen und der Arbeitspunkt / die Arbeitsfrequenz wird bestimmt. Es wird ein Wert gewählt, der unterhalb des Maximalwertes liegt, damit bei der Puls-Pausen-Modulation (PPM) ein ausgewogenes Verhältnis (ein/aus) eingestellt werden kann - in der Regel liegt dieses Verhältnis bei 70% "an" und 30% "aus". Ein zu hoher gewählter Wert, der aufgrund der Nutzung des Maximalwertes resultieren würde, hätte sehr kurze "ein"-Phasen und sehr lange "aus"-Phasen bzw. Zeiten zur Folge.

Somit werden über die PPM alle Belastungszustände des Schallkopfes 3 nachgeregelt und eine konstante und besonders gleichmäßige Leistungsabgabe ist möglich.

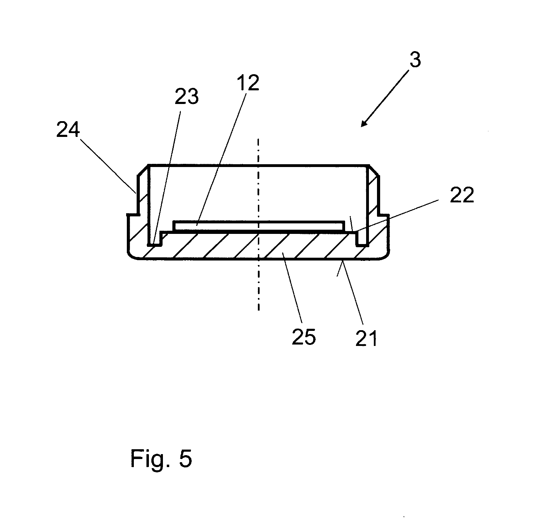

In Figur 5 ist ein Schallkopf 3 dargestellt, der als eine metallische Kappe 20 ausgebildet ist, deren außenliegenden Stirnfläche 21 die auf eine Hautpartie aufsetzbare Applikationsfläche bildet. Eine von der Stirnfläche 21 und einer der Stirnfläche 21 abgewandte Innenfläche 22 begrenzte Stirnwandung 25 weist von der Innenfläche 22 her eine umlaufende Nut 23 auf. Über einen rückwärtigen Ansatz 24 ist der Schallkopf 3 in das Gehäuse 2 einsetzbar.

Das Ultraschall-Wandlerelement 12, das als ein scheibenförmiges Piezoelement ausgebildet ist, ist an der Innenfläche 22 der Kappe 20 durch eine Klebung mit dieser fest verbunden.

Über einen am Schallkopf 3 angeordneten Temperatursensor 26 wird die Temperatur des Schallkopfes 3 ermittelt, so dass bei Überschreiten einer vorgegebenen Grenztemperatur die Leistung des Schallkopfes 3 reduziert werden kann.

## Patentansprüche

1. Ultraschallbehandlungsgerät (1) mit einer in einem Gehäuse (2) angeordneten Energieversorgung, einem Oszillator (11) und einem dem Oszillator (11) nachgeschalteten Treiber (14) mit nachfolgender Leistungsstufe (15), die mit einem Ultraschall-Wandlerelement (12) eines Schallkopfes (3) verbunden ist,
**dadurch gekennzeichnet,**
**dass** die Leistungsstufe (15) als ein Filter (16) ausgebildet ist, über den die Rechteckspannung des Treibers (14) in eine sinusförmige Betriebsspannung umwandelbar und die Impedanz des Treibers (14) an die Impedanz des Ultraschall-Wandlerelements (12) anpassbar ist,
**dass** der Oszillator (11) als ein von einem Mikrocontroller (10) gesteuerter programmierbarer Oszillator (11) ausgebildet ist,
**dass** in Verbindung mit einer Strommessung (19), die den dem Treiber (14) zugeführten Stromfluss misst, zur Ansteuerung des Ultraschall-Wandlerelements (12) eine optimale Resonanzfrequenz bei maximalem Stromfluss ermittelbar ist,
**dass** die ermittelten Werte in einem Speicher abspeicherbar und von dem Mikrocontroller (10) verarbeitbar sind, und
**dass** zur Leistungsregelung durch eine Puls-Pausen-Modulation zwischen dem programmierbaren Oszillator (11) und dem Treiber (14) ein von dem Mikrocontroller (10) ansteuerbares Gate (13) angeordnet ist, wobei ein gegenüber dem maximalen Stromwert reduzierter Stromsollwert (I_{Soll}) zur Leistungsregelung konstant gehalten werden kann.

2. Ultraschallbehandlungsgerät nach Anspruch 1,
**dadurch gekennzeichnet,**
**dass** das Gehäuse (2) als ein rohrförmiges Handstück ausgebildet ist, das zu einer Applikationsstelle hin von dem Schallkopf (3) verschlossen ist, an dessen Innenseite das Ultraschall-Wandlerelement (12) angeordnet ist.

3. Ultraschallbehandlungsgerät nach Anspruch 1 oder 2,
**dadurch gekennzeichnet,**
**dass** das Ultraschall-Wandlerelement (12) als ein scheibenförmiges Piezoelement ausgebildet ist.

4. Ultraschallbehandlungsgerät nach Anspruch 2 oder 3,
**dadurch gekennzeichnet,**
**dass** die Stirnwandung (25) des Schallkopfes (3) an der Innenseite eine umlaufende Nut aufweist.

5. Ultraschallbehandlungsgerät nach einem der Ansprüche 2 bis 5,
**dadurch gekennzeichnet,**
**dass** das Gehäuse (2) an seinem dem Schallkopf (3) abgewandten Ende einen das Gehäuse (2) verschließenden Touch-Schalter (18) aufweist.

6. Ultraschallbehandlungsgerät nach einem der Ansprüche 1 bis 5,
**dadurch gekennzeichnet,**
**dass** ein Temperatursensor (26) im Gehäuse (2) vorgesehen ist und
**dass** bei Überschreiten einer vorgebbaren Grenztemperatur die Leistung des Schallkopfes (3) reduzierbar ist.

7. Verfahren zum Betrieb eines Ultraschallbehandlungsgerätes (1), bei dem ein Ultraschall-Wandlerelement (12) eines Schallkopfes (3) von einem Oszillatorsignal eines Oszillators (11) über einen nachgeschalteten Treiber (14) erregt wird,
**dadurch gekennzeichnet,**
**dass** der Oszillator (11) von einem Mikrocontroller (10) gesteuert und programmiert wird,
**dass** zwischen dem programmierbaren Oszillator (11) und dem Treiber (14) ein von dem Mikrocontroller (10) ansteuerbares Gate (13) angeordnet ist, wobei nach dem Einschalten des Ultraschallbehandlungsgerätes (1) folgende Schritte durchgeführt werden:
a) Frequenzscan, bei dem ein vorgegebenes Frequenzband bei gleichzeitiger Messung des Stromflusses, der dem Treiber (14) zugeführt wird, durchfahren wird, wobei die ermittelten Werte in einem Arbeitsspeicher abgelegt werden,
b) Ermittlung des Maximums des Stromflusses und zugehöriger optimaler Resonanzfrequenz,
c) Betrieb des Ultraschall-Wandlerelements (12) mit der ermittelten optimalen Resonanzfrequenz bei einer Regelung der Schallleistung durch Puls-Pausen-Modulation des Gates (13) auf einen vorgegebenen konstanten Wert, wobei der dem Treiber zugeführte Stromfluss durch die Puls-Pausen-Modulation auf einem vorgegebenen, gegenüber dem maximalen Stromwert reduzierten Stromsollwert (I_{Soll}) konstant gehalten wird.

8. Verfahren nach Anspruch 7,
**dadurch gekennzeichnet,**
**dass** die Schritte a) bis c) in vorgegebenen Zyklen wiederholt werden.

9. Verfahren nach Anspruch 7 oder 8,
**dadurch gekennzeichnet,**
**dass** als optimale Resonanzfrequenz eine unterhalb der maximalen Resonanzfrequenz liegende Frequenz verwendet wird.

10. Verfahren nach einem der Ansprüche 7 bis 9,
**dadurch gekennzeichnet,**
**dass** über einen Temperatursensor (26) die Temperatur des Schallkopfes (3) ermittelt und bei Überschreiten einer vorgegebenen Grenztemperatur die Leistung des Schallkopfes (3) reduziert wird.

## Claims

1. An ultrasonic treatment device (1) having a housing (2) in which are arranged an energy supply, an oscillator (11) and a driver (14) connected downstream of the oscillator (11) and followed by a power stage (15) that is connected to an ultrasonic transducer element (12) of a sound head (3),
**characterized in that**
the power stage (15) is designed as a flter (16) via which the square-wave voltage of the driver (14) can be converted into a sinusoidal operating voltage and the impedance of the driver (14) can be adapted to the impedance of the ultrasonic transducer element (12), **In that** the oscillator (11) is designed as a programmable oscillator (11) that is corttrolled by a microcontroller (10),
**in that**, in conjunction with a current measurement device (19), which measures the current flow supplied to the driver (14), an optimum resonance frequency can be determined at maximum current flow in order to control the ultrasonic transducer element (12),
**in that** the values determined can be stored in a memory and can be processed by the microcontroller (10),
**in that** in order to regulate the power by means of pulse interval modulation a gate (13), which can be controlled by the microcontroller (10), is arranged between the programmable oscillator (11) and the driver (14), whereby a current setpoint (Iₛₑₜₚₒᵢₙₜ), which is reduced relative to the maximum current value, can be kept constant in order to regulate the power.

2. The ultrasonic treatment device according to Claim 1,
**characterized in that**
the housing (2) is designed as a tubular handpiece that, in the direction of an application site, is closed off by the sound head (3), on the inner side of which is arranged the ultrasonic transducer element (12).

3. The ultrasonic treatment device according to Claim 1 or 2,
**characterized in that**
the ultrasonic transducer device (12) is designed as a disc-shaped piezo element.

4. The ultrasonic treatment device according to Claims 2 or 3,
**characterized in that**
the end wall (25) of the sound head (3) has a circumferential groove on its inner side.

5. The ultrasonic treatment device according to any of the Claims 2 to 5,
**characterized in that**
on its end facing away from the sound head (3) the housing (2) has a touch switch (18) that closes off the housing (2).

6. The ultrasonic treatment device according to any of Claims 1 to 5,
**characterized in that**
a temperature sensor (26) is provided in the housing (2)
and **In that** when a settable limit temperature is exceeded, the pawer of the sound head (3) can be reduced.

7. A method for operating an ultrasonic treatment device (1), wherein an ultrasonic transducer element (12) of a sound head (3) is energized by an oscillator signal from an oscillator (11), via a downstream-connected driver (14),
**characterized in that**
the oscillator (11) is controlled and programmed by a microcontroller (10),
and **in that** a gate (13) that can be controlled by the microcontroller (10) is arranged between the programmable oscillator (11) and the driver (14), and the following steps are carried out when the ultrasonic treatment device (1) is switched on:
a) a frequency scan, in which a predetermined frequency band is scanned while simultaneously the current flow supplied to the driver (14) is measured, with the values determined being stored in a work memory
b) determination of the maximum current flow and of the associated optimum resonance frequency,
c) operation of the ultrasonic transducer element (12) at the determined optimum resonance frequency, with the sound power being regulated by pulse interval Modulation of the gate (13) at a predetermined constant value, and with the current flow to the driver being held constant by the pulse interval modulation at a predetermined current setpoint value (Iₛₑₜₚₒᵢₙₜ) that is reduced relative to the maximum current value.

8. The method according to Claim 7,
**characterized in that**
the steps a) to c) are repeated in predetermined cycles.

9. The method according to Claim 7 or 8,
**characterized in that**
a frequency below the maximum resonance frequency is used as the optimum resonance frequency.

10. The method according to any of Claims 7 to 9,
**characterized in that**
the temperature of the sound head (3) is determined via a temperature sensor (26) and the power of the sound head (3) is reduced when a predetermined limit temperature is exceeded.

## Revendications

1. Appareil de traitement à ultrasons (1), avec une alimentation en énergie disposée dans un boîtier (2), avec un oscillateur (11) et avec un excitateur (14) monté en aval de l'oscillateur (11) et suivi d'un étage de puissance (15) qui est relié à un élément transducteur à ultrasons (12) d'une tête acoustique (3),
**caractérisé,**
**en ce que** l'étage de puissance (15) est réalisé sous la forme d'un filtre (16), au moyen duquel la tension rectangulaire de l'excitateur (14) peut être transformée en une tension de fonctionnement sinusoïdale et l'impédance de l'excitateur (14) peut être adaptée à l'impédance de l'élément transducteur à ultrasons (12),
**en ce que** l'oscillateur (11) est réalisé sous la forme d'un oscillateur programmable (11) commandé par un microcontrôleur (1 0),
**en ce que**, en liaison avec un ampèremètre (19) qui mesure le flux de courant apporté à l'excitateur (14), une fréquence de résonance optimale peut être déterminée en présence d'un flux de courant maximal afin d'asservir l'élément transducteur à ultrasons (12),
**en ce que** les valeurs déterminées peuvent être enregistrées dans une mémoire et traitées par le microcontrôleur (10),
et **en ce qu'**une gâchette (13) pouvant être asservie par le microcontrôleur (10) est disposée entre l'oscillateur programmable (11) et l'excitateur (14) afin de réguler la puissance par une modulation d'intervalles d'impulsions, sachant qu'une valeur de consigne d'intensité (I_{consigne}), réduite par rapport à la valeur maximale d'intensité, peut être maintenue constante afin de réguler la puissance.

2. Appareil de traitementà ultrasons selon la revendication 1,
**caractérisé en ce que** le boîtier (2) est réalisé en forme d'un élément portatif tubulaire, qui est fermé en direction d'un point d'application par la tête acoustique (3) sur le côté intérieur de laquelle est disposé l'élément transducteur à ultrasons (12).

3. Appareil de traitement à ultrasons selon la revendication 1 ou 2,
**caractérisé en ce que** l'élément transducteur à ultrasons (12) est réalisé sous la forme d'un élément piézoélectrique en forme de disque.

4. Appareil de traitement à ultrasons selon la revendication 2 ou 3,
**caractérisé en ce que** la paroi frontale (25) de la tête acoustique (3) présente sur le côté intérieur une rainure périphérique.

5. Appareil de traitement à ultrasons selon l'une des revendications 2 à 5,
**caractérisé en ce que** le boîtier (2) présente, à son extrémité opposée à la tête acoustique (3), un interrupteur tactile (18) fermant le boîtier (2).

6. Appareil de traitement à ultrasons selon l'une des revendications 1 à 5,
**caractérisé en ce qu'**un capteur de température (26) est prévu dans le boltier (2), et **en ce que** la puissance de la tête acoustique (3) peut être réduite en cas de dépassement d'une température limite prescriptible.

7. Procédé d'exploitation d'un appareil de traitement à ultrasons (1), selon lequel un élément transducteur à ultrasons (12) d'une tête acoustique (3) est excité par un signal d'oscillateur d'un oscillateur (11) par l'intermédiaire d'un excitateur (14) monté en aval,
**caractérisé**
**en ce que** l'oscillateur (11) est commandé et programmé par un microcontrôleur (10), en ce qu'une gâchette (13) pouvant être asservie par le microcontrôleur (10) est disposée entre l'oscillateur programmable (11) et l'excitateur (14), sachant qu'à la suite de l'activation de l'appareil de traitement à ultrasons (1), les étapes suivantes sont exécutées:
a) balayage de fréquence, au cours duquel on balaye une plage prescrite de fréquences tout en mesurant le flux de courant qui est apporté à l'excitateur (14), sachant que les valeurs déterminées sont enregistrées dans une mémoire de travail,
b) détermination du maximum du flux de courant et de la fréquence de résonance optimale correspondante,
c) exploitation de l'élément transducteur à ultrasons (12) à la fréquence de résonance optimale déterminée, avec une régulation de la puissance acoustique par modulation d'intervalles d'impulsions de la gâchette (13) à une valeur constante prescrite, sachant que le flux de courant apporté à l'excitateur est maintent constant, par la Modulation d'intervalles d'impulsions, à une valeur de consigne d'intensité prescrite (I_{consigne}), réduite par rapporta la valeur maximale d'intensité.

8. Procédé selon la revendication 7,
**caractérisé en ce que** les étapes a) à c) sont répétées parcycles prédéfinis.

9. Procédé selon la revendication 7 ou 8,
**caractérisé en ce qu'**on utilise comme fréquence de résonance optimale une fréquence située en dessous de la fréquence de résonance maximale.

10. Procédé selon l'une des revendications 7 à 9,
**caractérisé en ce que** la température de la tête acoustique (3) est déterminée au moyen d'un capteur de température (26), et que la puissance de la tête acoustique (3) est réduite en cas de dépassement d'une température limite prescrite.
